# EUROPEAN PATENT APPLICATION

(11) **EP 4 417 053 A1**
(43) Date of publication of application: **21.08.2024**
(21) Application number: 23157395.7
(22) Date of filing: 17.02.2023
(51) Int. Cl.: A01N 25/02, A01N 37/02, A01N 37/10, A61K 8/34, A61K 8/365, A61K 8/368, A61K 8/37, A61K 8/39, A61Q 19/00, A01P 1/00

(54) **COMPOSITION COMPRISING P-METHOXYBENZOIC ACID IN THE FORM OF AN AQUEOUS SOLUTION**

(71) Applicant: Evident Ingredients GmbH, 22081 Hamburg (DE)
(72) Inventor: CAKIR, Nargiza, 22297 Hamburg (DE); SIETZEN, Malte, 25469 Halstenbek (DE); IBARRA, Fernando, 22395 Hamburg (DE); PREUSSE GARCIA, David, 20537 Hamburg (DE)
(74) Representative: Eisenführ Speiser

(57) **Abstract**

The invention relates to an aqueous composition in the form of a solution, the composition comprising p-methoxybenzoic acid sodium salt, levulinic acid sodium salt, and glycerol; the preparation of the composition; and the use of the composition.

## Description

The invention relates to a composition comprising p-methoxybenzoic acid, the preparation of the composition, and the use of the composition.

The manufacture of personal care products requires protection against microbial spoilage, since many of such products offer ideal growth conditions to a number of microorganisms. In contrast to food products, which are often consumed within days after opening the packaging, consumers expect personal care products to have a shelf life of at least six months after opening, as this is for most cosmetic items the period typically required for using up.

When the mass production and worldwide sale of personal care products became feasible in the middle of the 20th century, substances such as formaldehyde-releasers, phenols, and halocarbons were employed among others as such chemical preservatives. However, it has since been discovered that many of the substances as used over the last decades have harmful side-effects on the skin or other parts of the body. In extreme cases, this led to the general ban of certain preservatives (such as methyldibromoglutaronitrile) or to a dosage restriction to minimize the harmful effect (for example methylisothiazolinone). Another famous example are parabens, for which the allowed usage percentage has been drastically lowered in many jurisdictions. Thus, there continues to be a need for less harmful chemical preservatives.

One class of modern antimicrobial substances used extensively today are weak organic acids, such as benzoic acid, sorbic acid, levulinic acid, and p-methoxybenzoic acid (p-anisic acid). These compounds are known to be effective antimicrobials, whilst being mild on the skin. Disadvantages are the pH-restriction, as efficacy is limited to a formulation pH of about 4.5-6.5, as well as low solubility and thereby tendency to crystallize and become inactive.

In recent years, especially p-methoxybenzoic acid has raised high interest among formulators and consumers alike, as, in addition to the advantages listed above, the substance can be obtained from renewable sources and is thus considered to be an eco-friendly (sustainable) alternative to petrochemicals. However, the limited solubility of p-methoxybenzoic in water (only about 0.2 g/L) prevents formulation of aqueous concentrates of p-methoxybenzoic acid. Such concentrates are required because the concentration required to achieve an antimicrobial effect in a product is substantially higher. In order for such concentrates to be suitable for the production of cosmetics, the concentrates need to be pumpable and need to be free from any precipitation of constituent. These concentrates also need to retain their colour when stored for an extended time period, as they should not contribute to the colour of the cosmetic into which they are formulated.

This problem is addressed in EP 1 709 955 A1, according to which polyols, polyglyceryl esters, sorbitan esters, and organic acids and salts thereof are suggested as solubilizers, to be able to prepare a liquid and concentrated mixture containing p-methoxybenzoic acid. However, extensive use of solubilizers adds to the cost of the concentrates and furthermore prevents the preparation of concentrates that are suitable for a wide range of different types of cosmetics. Also, EP 1 709 955 A1 suggests to use a process that requires one to carefully avoid any crystallization during each step of production, and this process is difficult to carry out and requires potentially longer production time. Moreover, and as shown below, the mixtures exemplified in EP 1 709 955 A1 cannot be prepared as clear and long term-stable as well as concentrated solutions. Moreover, the process for the preparation of the concentrate composition goes through a process step at high pH value, and the heat of neutralisation may lead to decomposition products being present in the concentrate composition, such as alpha-angelicalactone, which is a known decomposition product of levulinic acid.

Also, some concentrates that are commercially available and that contain p-methoxybenzoic acid sodium salt and glycerol may be turbid rather than clear solutions, and may turn brown when stored for an extended period of time.

EP 1 325 731 A1 teaches solutions of p-methoxybenzoic acid in a polyol and a cationic surfactant. However, surfactants may form micelles and thus encapsulate and deactivate the acid. Furthermore, surfactants are often incompatible with emulsifiers, which are commonly found as functional ingredients in a number of personal care products.

The application of solid sodium p-methoxybenzoate in conjunction with sodium levulinate is described in EP 3 714 694 A1. However, it is generally recognized that the handling of powdery substances carries the risk of dust explosions, as well as the risk that manufacturing personnel may come into direct contact with the substances through inhalation. In addition, solid raw materials cannot be pumped and are thus more difficult to weigh, therefore often deemed unfavourable. So, whilst the problem of low solubility is solved when using solid sodium p-methoxybenzoate, safety deficiencies and process disadvantages persist.

US 2004/0167195 A1 teaches a cosmetic composition comprising 1 to 30 % by weight of p-methoxybenzoic acid and up to 50 % by weight of one or more polyols, such as glycerol. Exemplified compositions further comprise citric acid and sodium hydroxide, resulting in an overall acidic pH value of about 5.2. At such acidic conditions, p-methoxybenzoic acid is not primarily present as salt, but in the form of the antimicrobially active acid form, which however does not form long-term stable aqueous solutions.

The object of the present invention was to find a composition in the form of a concentrated solution. The composition should comprise, apart from p-methoxybenzoic acid or its sodium salt, only those constituents that are required to obtain a clear, pumpable concentrate, in as low as possible amounts. Moreover, the composition should be stable for an extended period of time, without forming side products or changing colour, so as to not make the formulation of cosmetics difficult.

It has surprisingly been found that this object could be solved in accordance with a first aspect of the present invention with a liquid composition comprising, based on the total weight of the composition,
a) 3 to 8 % by weight of the sodium salt of p-methoxybenzoic acid, calculated as free p-methoxybenzoic acid,
b) 5 to 30 % by weight of water,
c) 10 to 20 % by weight of the sodium salt of levulinic acid, calculated as free levulinic acid,
d) 40 to 70 % by weight of glycerol,
wherein the pH value of the composition is in a range of from 7.0 to 9.0, and the composition is in the form of a solution.

Moreover, and in a second aspect of the invention, the composition according to the first aspect can surprisingly be prepared as a solution in a process comprising
a. stirring glycerol in an amount of 50 to 90 % of the total required amount, water, and at least part of the levulinic acid, in a vessel;
b. adding the p-methoxybenzoic acid to the vessel;
c. continuing stirring;
d. adding the remaining amount of glycerol to the vessel;
e. gradually adding aqueous sodium hydroxide to the vessel, such that
   i. the temperature does not exceed 45°C, and
   ii. the pH value does not exceed 9.0;
f. continuing stirring, to give a homogeneous solution, and cooling the solution to below 30°C;
g. obtaining the composition, optionally after adjusting the pH value with a dilute aqueous solution of levulinic acid, or with a dilute aqueous solution of sodium hydroxide.

Also, the composition of the first aspect is in a third aspect of the invention used for the antimicrobial stabilization of products pertaining to personal care, cosmetic, dermatology, hair care, or oral care.

### Detailed description

In a first aspect, the invention relates to a liquid composition comprising, based on the total weight of the composition,
a) 3 to 8 % by weight of the sodium salt of p-methoxybenzoic acid, calculated as free p-methoxybenzoic acid,
b) 5 to 30 % by weight of water,
c) 10 to 20 % by weight of the sodium salt of levulinic acid, calculated as free levulinic acid,
d) 40 to 70 % by weight of glycerol.

According to the invention, the pH value of the composition is in a range of from 7.0 to 9.0, and the composition is in the form of a solution.

The composition according to the invention is a clear, typically light yellow solution, i.e. constituents a) and c) are present in a moleculardisperse fashion in the constituents b) and d). It can therefore be used in cosmetic products without undesirably contributing to the colour of the cosmetic. Also, and in contrast to the procedure of EP 1 709 955 A1, the process for the preparation of the composition does not result in high temperature through neutralisation of the p-methoxybenzoic and levulinic acids, nor are the constituents of the composition during preparation exposed to any high pH value. Therefore, the formation of decomposition products is avoided. Moreover, the composition because of the favourable ease of preparation has less of a strong odour, as compared to commercially available concentrates comprising the sodium salt of p-methoxybenzoic acid.

Moreover, the relatively high amount of glycerol present in the composition allows the formulator of a cosmetic to take advantage of the humectant properties of glycerol, as well as the strengthening of the protecting barrier function of the *stratum corneum.* Glycerol furthermore has a calming and anti-irritant effect on the skin. The composition of the invention also contains a relatively low amount of levulinic acid, lower than in the compositions exemplified in EP 1 709 955 A1. This, in particular, contributes to the compositions of the invention having less of a tendency to yellow upon storage. Also, the higher amount of glycerol present, as compared to commercially available compositions comprising p-methoxybenzoic acid, reduces the composition's freeze point and the composition can therefore also be held and handled at lower temperatures.

According to the invention, it is preferred that the amount of the sodium salt of p-methoxybenzoic acid in the composition, calculated as free p-methoxybenzoic acid, is in a range of from 3.5 to 7 % by weight, more preferably in a range of from 4 to 6 % by weight, most preferably in a range of from 4.5 to 5.5 % by weight, such as about 5 % by weight.

These preferred compositions are not only advantageous in terms of storage stability, they also provide a relatively high amount of p-methoxybenzoic acid to the cosmetic to be antimicrobially stabilized.

Moreover, it is preferred that the amount of water in the composition is in a range of from 7.5 to 23 % by weight, more preferably in a range of from 10 to 19 % by weight and most preferably in a range of from 12 to 17 % by weight, such as about 14.5 % by weight.

These preferred compositions contain an optimal amount of water and, whilst being stable as clear solution when stored, contain only the minimum amount of water, i.e. do not unnecessarily dilute the composition.

Also, it is preferred that the amount of the sodium salt of levulinic acid in the composition, calculated as free levulinic acid, is in a range of from 12 to 18 % by weight, more preferably in a range of from 13 to 17 % by weight and most preferably in a range of from 14 to 16 % by weight, such as about 15 % by weight.

Such preferred compositions contain a relatively low amount of levulinic acid, as compared to the teaching of EP 1 709 955 A1.

Additionally, it is preferred that the amount of glycerol in the composition is in a range of from 50 to 68 % by weight, preferably in a range of from 60.05 to 66 % by weight and more preferably in a range of from 61.5 to 64 % by weight, such as about 62 % by weight.

These preferred compositions contain a relatively high amount of glycerol, with the associated advantages in the resulting cosmetics, such as skin-friendliness.

Moreover, it is preferred that the pH value of the composition is in the range of from 7.0 to 8.5, preferably 7.0 to 8.0.

These preferred compositions have a pH value that ensures a particularly good storage stability.

Preferred compositions are listed in the following table (amounts in wt.%, based on the total weight of the composition):

| Constituent | Preferably | More preferably | Most preferably | In particular |
|---|---|---|---|---|
| p-Methoxybenzoic acid¹ | 3.5 to 7 | 4 to 6 | 4.5 to 5.5 | about 5 |
| Water | 7.5 to 23 | 10 to 19 | 12 to 17 | about 14.5 |
| Levulinic acid² | 12 to 18 | 13 to 17 | 14 to 16 | about 15 |
| Glycerol | 50 to 68 | 60.05 to 66 | 61.5 to 64 | about 62 |
| pH | 7.0 to 8.5 | 7.0 to 8.0 | 7.0 to 8.0 | about 7.5 |

| | | | | |
|---|---|---|---|---|
| ¹ Present in the composition as sodium salt, calculated as free p-methoxybenzoic acid. ² Present in the composition as sodium salt, calculated as free levulinic acid. | | | | |

The composition of the invention preferably has a colour of 6 or lower on the Gardner scale.

The composition of the invention preferably contains less than 5 % by weight, based on the total weight of the composition, of compounds that are other than 1) p-methoxybenzoic acid and the sodium salt thereof, 2) water, 3) levulinic acid and the sodium salt thereof, and 4) glycerol. More preferably, the amount of the compounds is less than 3 % by weight, most preferably, the amount of the compounds is less than 2 % by weight, in particular, the amount of the compounds is less than 1 % by weight. Such compounds and their amounts are easily determined by HPLC analysis.

Also, the composition preferably contains less than 2 % by weight, based on the total weight of the composition, of alpha-angelicalactone. More preferably, the amount of alpha-angelicalactone is less than 1 % by weight, most preferably the amount of alpha-angelicalactone is less than 0.5 % by weight, in particular, the amount of alpha-angelicalactone is less than 0.2 % by weight, less than 0.1 % by weight, or even less than 0.05 % by weight, each based on the weight of the composition. The amount of alpha-angelicalactone can be easily determined by HPLC.

In a second aspect, the invention relates to a process for the preparation of the composition according to the first aspect. The process comprises
a. stirring glycerol in an amount of 50 to 90 % of the total required amount, water, and at least part of the levulinic acid, in a vessel;
b. adding the p-methoxybenzoic acid to the vessel;
c. continuing stirring;
d. adding the remaining amount of glycerol to the vessel;
e. gradually adding aqueous sodium hydroxide to the vessel, such that
   i. the temperature does not exceed 45°C, and
   ii. the pH value does not exceed 9.0;
f. continuing stirring, to give a homogeneous solution, and cooling the solution to below 30°C;
g. obtaining the composition, optionally after adjusting the pH value with a dilute aqueous solution of levulinic acid, or with a dilute aqueous solution of sodium hydroxide.

This process allows for the preparation of the composition according to the first aspect, without the disadvantages of the process of EP 1 709 955 A1 (such as tendency of the compositions to yellow upon storage).

The process of the invention converts the starting materials p-methoxybenzoic acid and levulinic acid into their respective sodium salts.

Preferably, and in order to improve circulation when stirring, the process according to the second aspect of the invention is performed in a cylindrical vessel.

In a third aspect, the invention relates to the use of the composition of the first aspect for antimicrobial stabilization of products pertaining to personal care, cosmetic, dermatology, hair care, or oral care.

Use concentrations of the compositions according to the first aspect should be between 2.0 % by weight and 5.0 % by weight of the cosmetic, depending on the pH of the cosmetic formulation, and any other antimicrobials present therein. Preservative efficacy testing is employed to find a suitable application concentration for producing a safe cosmetic product.

In a preferred embodiment of the use of the third aspect, the product is an emulsion, shampoo, body wash, micellar water, toothpaste, mouthwash, or a decorative cosmetic.

Furthermore, in a preferred embodiment of the use of the third aspect, the products is a lipstick, a mascara, or an eye shadow.

In accordance with the present invention, p-methoxybenzoic (anisic) acid is in the composition kept in solution by using a relatively low amount of levulinic acid, namely by using a relatively high amount of glycerol. This is possible because the present invention uses a different method for preparing the composition, as compared to the disclosure of EP 1 709 955 A1.

Mixtures prepared according to the present invention are not prone to crystallization of p-methoxybenzoic acid or its sodium salt even if kept at low temperatures for an extended period of time. Even when freezing of the mixture occurs at low temperatures, the solidification is completely reversible and the product regains its transparent and homogenous character after thawing. In addition, mixtures prepared according to the present invention are superior to those described in the state of the art in the following ways:
o Through the reduction of the required amount of levulinic acid as solubilizing agent, costs can be saved and lower amounts of chemical substances are employed, making the mixtures prepared according to this invention more sustainable and cost effective.
∘ Mixtures prepared according to the present invention do not exceed pH 9.0 during manufacture. This is desirable since sodium levulinate is known to undergo a colour change at alkaline conditions. Furthermore, decomposition reactions are always possible at extreme pH conditions, such as enolate formation at the sodium levulinate C-5 atom and subsequent nucleophilic attacks, leading to unwanted side products. The process of the invention saves time, energy and uses less raw materials, and is therefore an important step to the respectful use of resources. Since this results in cost saving in the production step, it offers a saving potential for producers and ultimately also consumers of cosmetic products worldwide.

Mixtures prepared according to the present invention perform similar in preservative efficacy tests when compared to commercially available concentrates, when judged based to the same amount of p-methoxybenzoic acid introduced into the cosmetic.

The advantages of the invention become apparent from the following examples, where all percentages are by weight (unless stated otherwise).

### Examples:

In the following experiments, attempts were made to prepare compositions comprising p-methoxybenzoic acid, levulinic acid, glycerol and water in the required proportions. In Experiment A, the preparation method of EP 1 709 955 A1 was used. Experiment B shows results with the preparation method according to the present invention.

### A. Attempted repeat of EP 1 709 955 A1

The following examples were performed in accordance with the disclosure of EP 1 709 955 A1 (see para. [0018]). First, p-methoxybenzoic acid was dissolved in the polyol and, then, the sodium hydroxide solution is added. Finally, and where applicable, the pH-value was adjusted with levulinic acid. Each experiment was performed two or three times. The individual preparations are listed in Table 1 (in this table as well as Table 2, the amounts are indicated as used in the preparation method: in order to calculate the amount of water as present in the final composition, the amount of water included via the aqueous sodium hydroxide needs to be added to the value for "added water", and, further, the water produced from neutralisation of the p-methoxybenzoic acid and the levulinic acid by the sodium hydroxide also needs to be added).

**Table 1**

| No. | p-methoxybenzoic acid (wt.%) | levulinic acid (wt.%) | sodium hydroxide (wt.%)⁵ | polyol (wt.%) | added water (wt.%) | pH value | Solution⁴ |
|---|---|---|---|---|---|---|---|
| I | 5 | 20 | 16.5 | 50² | 8.5 | 7.41 | No |
| II | 5 | 25 | 19.9 | 50² | 0.1 | 7.32 | No |
| III | 5 | 30 | 24.4 | 41.3² | - | 7.43 | No |
| IV | 5 | 15 | 13 | 60² | 7 | 7.06 | No |
| V | 5 | 20 | 16.5 | 20² | 38.5 | 7.11 | No |
| VI | 5 | 20 | 16.5 | 58.5² | - | 8.26 | No |
| VII | 5 | 30 | 23.4 | 20² | 21.6 | 7.37 | No |
| VIII¹ | 5 | - | 15 (10%) | 30² | 50 | 11.60 | No |
| IX¹ | 5 | - | 14 (10%) | 31.5³ | 49.9 | 7.54 | No |
| X¹ | 5 | 21 | 19 (45%) | 50² | 5 | 7.61 | No |
| XI¹ | 7.5 | 22.5 | 21.5 (45%) | 25² | 23.5 | 7.42 | No |

| | | | | | | | |
|---|---|---|---|---|---|---|---|
| ¹ Nos VIII to XI correspond to solutions 1 to 4 as exemplified in EP 1 709 955 A1. ² Prepared with glycerol. ³ No 12 was prepared using polyglycerol-10 laurate. ⁴ Can a stable solution be formed in a reproducible manner? ⁵ As 50 wt.% aqueous solution unless stated otherwise. | | | | | | | |

It follows from the examples listed in Table 1 that, when preparing these compositions according to the method of EP 1 709 955 A1, no solutions comprising 40 wt.% or more of glycerol were obtained. Instead, precipitates formed in each of the experiments shown in Table 1.

### B. Examples according to the present invention

The preparation process according to the second aspect of the invention was conducted as follows, to give compositions as solutions:
- About 75 % of the total amount of glycerol, the water and the levulinic acid were placed into a suitable stirring vessel, and were stirred.
- Then, the p-methoxybenzoic acid was added and the mixture was stirred for about five minutes. A large part of the p-methoxybenzoic acid remained undissolved.
- The remaining amount of glycerol was added.
- The sodium hydroxide was added, as 50 wt.% solution in water. This addition was made batch wise. In this step, the reaction mixture was kept at a temperature of at most 45° C, by cooling. Also, the addition was made such that the pH value did not exceed 9.0. A homogeneous, clear mixture without solid particles was formed, which was then stirred until having cooled to room temperature.
- The pH value was optionally adjusted by using a small amount of dilute levulinic acid or dilute sodium hydroxide.

**Table 2**

| No. | p-methoxybenzoic acid (wt.%) | levulinic acid (wt.%) | sodium hydroxide (wt.%)¹ | glycerol (wt.%) | Added water (wt.%) | pH value | Clear solution⁴ |
|---|---|---|---|---|---|---|---|
| I | 5 | 15 | 13 | 65 | 2 | 7.5 | Yes |
| II | 5 | 15 | 13 | 60 | 7 | 7.5 | Yes |
| III | 5 | 15 | 13 | 55 | 12 | 7.5 | Yes |

| | | | | | | | |
|---|---|---|---|---|---|---|---|
| ¹ As 50 wt.% aqueous solution. | | | | | | | |

The results from the experiments shown in Table 2 show that, when preparing compositions with the method of the present invention, solutions are formed. For instance, looking at composition IV in Table 1, which when produced in accordance with the method of EP 1 709 955 A1 does not form a solution, can be formulated as a solution when using the method of the present invention (see composition II in Table 2).

## Claims

1. Liquid composition comprising, based on the total weight of the composition,
a) 3 to 8 % by weight of the sodium salt of p-methoxybenzoic acid, calculated as free p-methoxybenzoic acid,
b) 5 to 30 % by weight of water,
c) 10 to 20 % by weight of the sodium salt of levulinic acid, calculated as free levulinic acid,
d) 40 to 70 % by weight of glycerol,
wherein the pH value of the composition is in a range of from 7.0 to 9.0, and wherein the composition is in the form of a solution.

2. The composition of claim 1, wherein the amount of the sodium salt of p-methoxybenzoic acid, calculated as free p-methoxybenzoic acid, is in a range of from 3.5 to 7 % by weight, preferably in a range of from 4 to 6 % by weight, more preferably in a range of from 4.5 to 5.5 % by weight, such as about 5 % by weight.

3. The composition of claim 1 or claim 2, wherein the amount of water is in a range of from 7.5 to 23 % by weight, preferably in a range of from 10 to 19 % by weight and more preferably in a range of from 12 to 17 % by weight, such as about 14.5 % by weight.

4. The composition of any of the preceding claims, wherein the amount of the sodium salt of levulinic acid, calculated as free levulinic acid, is in a range of from 12 to 18 % by weight, preferably in a range of from 13 to 17 % by weight and more preferably in a range of from 14 to 16 % by weight, such as about 15 % by weight.

5. The composition of any of the preceding claims, wherein the amount of glycerol is in a range of from 50 to 68 % by weight, preferably in a range of from 60.05 to 66 % by weight and more preferably in a range of from 61.5 to 64 % by weight, such as about 62 % by weight.

6. The composition of any of the preceding claims, wherein the pH value of the composition is in the range of from 7.0 to 8.5, preferably 7.0 to 8.0.

7. The composition of any of the preceding claims, wherein the colour of the composition is 6 or lower on the Gardner scale.

8. The composition of any of the preceding claims, containing less than 5 % by weight, based on the total weight of the composition, of compounds that are other than 1) p-methoxybenzoic acid and the sodium salt thereof, 2) water, 3) levulinic acid and the sodium salt thereof, and 4) glycerol,
preferably wherein the amount of the compounds is less than 3 % by weight,
more preferably wherein the amount of the compounds is less than 2 % by weight,
most preferably wherein the amount of the compounds is less than 1 % by weight.

9. The composition of any one of claims 1 to 7, containing less than 2 % by weight, based on the total weight of the composition, of alpha-angelicalactone,
preferably wherein the amount of alpha-angelicalactone is less than 1 % by weight,
more preferably wherein the amount of alpha-angelicalactone is less than 0.5 % by weight,
most preferably wherein the amount of alpha-angelicalactone is less than 0.2 % by weight.

10. A process for the preparation of the composition according to any of the preceding claims, comprising
a. stirring glycerol in an amount of 50 to 90 % of the total required amount, water, and at least part of the levulinic acid, in a vessel;
b. adding the p-methoxybenzoic acid to the vessel;
c. continuing stirring;
d. adding the remaining amount of glycerol to the vessel;
e. gradually adding aqueous sodium hydroxide to the vessel, such that
i. the temperature does not exceed 45°C, and
ii. the pH value does not exceed 9.0;
f. continuing stirring, to give a homogeneous solution, and cooling the solution to below 30°C;
g. obtaining the composition, optionally after adjusting the pH value with a dilute aqueous solution of levulinic acid, or with a dilute aqueous solution of sodium hydroxide.

11. Use of the composition of any of claims 1 to 9, for the antimicrobial stabilization of products pertaining to personal care, cosmetic, dermatology, hair care, or oral care.

12. The use of claim 11, wherein the product is an emulsion, shampoo, body wash, micellar water, toothpaste, mouthwash, or a decorative cosmetic,
preferably wherein the product is a lipstick, a mascara, or an eye shadow.

13. The use of claim 11 or claim 12, wherein the product is an o/w or a w/o emulsion.
